# EUROPÄISCHE PATENTANMELDUNG

(11) **EP 4 227 292 A1**
(43) Veröffentlichungstag der Anmeldung: **16.08.2023**
(21) Anmeldenummer: 22156145.9
(22) Anmeldetag: 10.02.2022
(51) Int. Cl.: C07C 263/10, C07C 263/20, C07C 265/14

(54) **VERFAHREN ZUR HERSTELLUNG VON ISOCYANATEN**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Die Erfindung betrifft ein Verfahren zur Herstellung von Isocyanaten durch Umsetzung von mindestens einem Amin oder dessen Salz mit einem stöchiometrischen Überschuss an Phosgen in kondensierter Phase in Gegenwart eines Lösemittels, umfassend die Schritte: A) Übergang in eine Heißphosgenierung und B) Austreiben von verbliebenem Phosgen und/oder Chlorwasserstoff, dadurch gekennzeichnet, dass die Umsetzung chargenweise in mindestens zwei parallel angeordneten Reaktoren erfolgt und dass Schritt A) und/oder Schritt B) in mindestens zwei der parallel angeordneten Reaktoren asynchron erfolgen.

## Beschreibung

Die Erfindung betrifft ein Verfahren zur Herstellung von Isocyanaten durch Umsetzung entsprechender Amine mit Phosgen in inerten Lösemitteln.

Isocyanate sind wichtige Grundstoffe der chemischen Industrie. Sie werden oft in großen Mengen hergestellt. Vor allem Di- und Polyisocyanate werden hauptsächlich als Ausgangsstoffe für die Herstellung von Polyurethanen eingesetzt. Ihre großtechnische Herstellung erfolgt in der Regel in guten Ausbeuten durch die Phosgenierung der entsprechenden Amine, Amin-Hydrochloride und/oder Amin-Carbonate mit einem Überschuss an Phosgen. Als ein Zwischenprodukt entsteht dabei das entsprechende Carbaminsäurechlorid, welches dann unter Abspaltung von Chlorwasserstoff in das Isocyanat überführt wird.

Als Lösemittel für die Reaktion haben sich in der Praxis Chlorbenzol oder o-Dichlorbenzol etabliert, die sich weitgehend inert verhalten und gut geeignet sind, überschüssiges Phosgen wiederzugewinnen und von Chlorwasserstoff zu trennen. Es ist jedoch auch möglich, andere, unter den Reaktionsbedingungen inerte Lösemittel zu verwenden.

Das Prozessabgas, das im Wesentlichen aus Phosgen, Chlorwasserstoff und Lösemittel besteht, muss behandelt werden, um eine Freisetzung von Phosgen zu vermeiden. Um die Wirtschaftlichkeit der Verfahren zu erhöhen, ist es überdies zweckmäßig, das Prozessabgas, welches im Wesentlichen aus Phosgen, Chlorwasserstoff und Lösemittel besteht aufzubereiten, so dass zumindest ein Teil dieser Bestandteile, weiter- oder wiederverwendet werden kann. Besonders vorteilhaft ist es, das Phosgen weiter- oder wiederzuverwenden.

Die US2007/0249859A1 beschreibt eine mindestens 2-stufige Sequenz von Absorptionsschritten, wobei die Sequenz mindestens einen isothermen Absorptionsschritt und mindestens einen adiabatischen Absorptionsschritt umfasst und das auf diese Weise gewonnenen Phosgen in die Phosgenierungsreaktion zurückgeführt wird.

In DE102008009761A1 wird der Prozessabgasstrom zunächst teilweise kondensiert und das flüssige Kondensat in einer Strippkolonne aufbereitet. Dort wird am Sumpf ein flüssiger Lösemittelstrom erhalten und am Kopf ein Gasstrom, der zusammen mit den zuvor nicht kondensierten Anteilen in eine Absorption geleitet wird. Dort wird das enthaltene Phosgen in einem Lösemittel absorbiert und die erhaltene Phosgen-Lösung wird, gegebenenfalls nach Anreicherung mit weiterem Phosgen, wieder in der Phosgenierungsreaktion eingesetzt.

Auf eine destillative Trennung des Prozessabgases setzt die US2018/0044179A1. Hier wird der Prozessabgasstrom in eine Destillationskolonne geleitet, an deren Sumpf ein phosgenhaltiger Strom entnommen wird. Am Kopf der Kolonne wird ein im Wesentlichen aus Chlorwasserstoff bestehender Strom entnommen, verdichtet und dabei teilweise kondensiert. Der kondensierte Anteil wird entspannt und als Rücklauf am Kopf der Kolonne aufgegeben.

Während Phosgen also häufig in den gleichen Prozess rezykliert wird, bieten sich für Chlorwasserstoff andere Verwertungsmöglichkeiten an. Er kann beispielsweise einfach als wässrige Lösung, also als Salzsäure genutzt oder vermarktet werden. Alternativ kann er katalytisch oder elektrochemisch zu Chlor oxidiert oder in der Oxichlorierung von Ethylen zu Ethylendichlorid genutzt werden.

Die WO2008049783A1 beschreibt ein kontinuierliches Verfahren zur Herstellung von Isocyanaten, bei dem das Mischen der Eduktströme und/oder die Umsetzung des dabei erhaltenen Reaktionsgemisches in mindestens zwei parallel geschalteten Strängen erfolgt, so dass bei Teillast-Fahrweise einzelne Stränge abgeschaltet werden und die in Betrieb befindlichen Stränge weiterhin im optimalen Bereich laufen.

Viele Isocyanate werden in großen Mengen hergestellt und dabei sind kontinuierliche Verfahren bevorzugt. Isocyanate, die in kleineren Mengen hergestellt werden, werden in der Regel durch chargenweise Flüssigphasenphosgenierung hergestellt, da der Aufwand für eine Umstellung auf ein kontinuierliches Verfahren sehr groß ist und sich angesichts der geringen herzustellenden Mengen nicht bezahlt macht. In einigen Fällen werden auch hybride Prozesse betrieben, in denen eine chargenweise Reaktionsführung mit einer kontinuierlich betriebenen, stromabwärtsgelegenen Aufbereitung der hergestellten Isocyanate kombiniert wird. Dies erfordert die Installation von Pufferbehältern, in die die Reaktionsprodukte abgelassen werden und aus denen dann die kontinuierliche Aufarbeitung gespeist wird.

Auch für die chargenweise Herstellung von Isocyanaten ist eine Behandlung des Abgases und nach Möglichkeit auch die Rückgewinnung von Phosgen aus diesem Abgas wünschenswert. Es ergeben sich allerdings Probleme, auf die im Stand der Technik bisher nicht eingegangen wurde. So sind die Massenströme an Prozessabgas sowie dessen Zusammensetzung je nach Betriebszustand stark schwankend und während es für flüssige Produktströme relativ einfach möglich ist, einen Puffertank zu installieren, und so für einen gleichmäßigen Zulauf-Strom zur Aufarbeitung zu sorgen, ist dies für das Prozessabgas aufgrund der großen Volumina sowie der toxischen und korrosiven Bestandteile nicht einfach möglich. Es ergeben sich während einer chargenweise durchgeführten Phosgenierung Zeiten mit sehr hohen Massenströmen an Prozessabgas. Um nun beispielsweise in einer Phosgenabsorption gemäß DE102008009761A1 oder US2007/0249859A1 eine unvollständige Absorption von Phosgen und damit den Durchbruch von Phosgen mit dem HCl-Abgas der Phosgenabsorption sicher zu vermeiden, müsste die Absorptionskolonne und auch der Massenstrom an Lösemitteln für diese Spitzen mit sehr hohen Massenströmen an Prozessabgas ausgelegt werden. Folglich wäre die Absorptionskolonne außerhalb dieser Spitzenlasten überdimensioniert, was wiederum weitere Probleme nach sich ziehen würde.

US4233267A beschreibt ein kombiniertes System aus einem Batch-Reaktor und einer kontinuierlichen Destillationsapparatur, wobei die flüchtigen Reaktionsprodukte zunächst kondensiert und in einem Kondensatbehälter zwischengelagert werden, um von dort aus im Wesentlichen unabhängig von den Betriebsparametern des Batch-Reaktors in die kontinuierlich betriebene Kolonne gespeist zu werden. Für Phosgenier-Prozesse ergeben sich bei dieser Vorgehensweise verschiedene Nachteile. Einerseits erfordert die Kondensation von Phosgen oder gar HCl sehr niedrige Temperaturen. Andererseits erhöht eine solche Vorgehensweise aufgrund der notwendigen Pufferung zwangsläufig die Menge an in der Anlage befindlichem Phosgen.

Aufgabe der Erfindung war es also ein verbessertes Verfahren bereitzustellen, mit dem sich auch bei chargenweiser Isocyanatherstellung durch Phosgenierung der Amine eine Prozessabgasaufbereitung effizient betreiben lässt und das dabei zurückgewonnene Phosgen möglichst wieder im Phosgenierprozess eingesetzt werden kann.

Diese Aufgabe konnte gelöst werden durch ein Verfahren zur Herstellung von Isocyanaten durch Umsetzung von mindestens einem Amin oder dessen Salz mit einem stöchiometrischen Überschuss an Phosgen in kondensierter Phase in Gegenwart eines Lösemittels, umfassend die Schritte:
A) Übergang in eine Heißphosgenierung und
B) Austreiben von verbliebenem Phosgen und/oder Chlorwasserstoff,
dadurch gekennzeichnet, dass die Umsetzung chargenweise in mindestens zwei parallel angeordneten Reaktoren erfolgt und dass Schritt A) und/oder Schritt B) in mindestens zwei der parallel angeordneten Reaktoren asynchron erfolgen.

Für das erfindungsgemäße Verfahren können sowohl Mono- als auch Polyamine, also Amine mit zwei oder mehr Aminogruppen je Molekül eingesetzt werden. Bevorzugt werden Diamine eingesetzt.

Beispiele für bevorzugte Diamine sind Diaminotoluol (TDA), insbesondere 2,4-Diaminoluol und 2,6-Diaminotoluol, Diaminodimethylbenzol, Diaminonaphthalin, insbesondere 1,5-Diaminonaphthalin (NDA), Diaminobenzol, insbesondere 1,4-Diaminobenzol (pPDI), Diaminodiphenylmethan (MDA), insbesondere 2,2'-Diaminodiphenylmethan, 2,4'-Diaminodiphenylmethan und 4,4'-Diaminodiphenylmethan, 1,4-Diaminobutan, 1,5-Diaminopentan (PDA), 1,6-Diaminohexan (HDA), 1,11-Diaminoundecan, 1-Amino-3,5,5-trimethyl-5-aminomethylcyclohexan (IPDA), Bis(p-aminocyclohexyl)methan (PACM), 1,5-Diamino-2-methylpentan, 2,5-Diamino-2,5-dimethylhexan, 1,4-Diaminocyclohexan, Hexahydrotoluylendiamin (H6TDA), insbesondere 2,4-Hexahydrotoluylendiamin, 2,6-Hexahydrotoluylendiamin, 1,3-Bis(aminomethyl)benzol (m-XDA), 1,4-Bis(aminomethyl)benzol (p-XDA), Bis(aminomethyl)cyclohexan (H6-XDA), Tetramethylxylylendiamin (TMXDA), Bis(aminomethyl)norbornan (NBDA), Neopentandiamin, 2,4,4-Trimethylhexamethylendiamin, 2,2,4-Trimethylhexamethylendiamin und Mischungen davon.

Beispiele für besonders bevorzugte Amine sind 1,5-Diaminonaphthalin (NDA), 1,4-Diaminobenzol (pPDA), 1,5-Diaminopentan (PDA), 1,6-Diaminohexan (HDA), 1-Amino-3,5,5-trimethyl-5-aminomethylcyclohexan (IPDA), Bis(p-aminocyclohexyl)methan (PACM), 1,5-Diamino-2-methylpentan, 2,5-Diamino-2,5-dimethylhexan, 1,4-Diaminocyclohexan, Hexahydrotoluylendiamin (H6TDA), 1,3-Bis(aminomethyl)benzol (m-XDA), 1,4-Bis(aminometyhl)benzol (p-XDA), Bis(aminomethyl)cyclohexan (H6-XDA), Bis(aminomethyl)norbornan (NBDA) und Mischungen davon.

Beispiele für ganz besonders bevorzugte Amine sind 1,5-Diaminonaphthalin (NDA), 1,4-Diaminobenzol (pPDA), 1,5-Diaminopentan (PDA), Bis(p-aminocyclohexyl)methan (PACM), Hexahydrotoluylendiamin (H6TDA), 1,3-Bis(aminomethyl)benzol (m-XDA), Bis(aminomethyl)norbornan (NBDA) und Mischungen davon.

Beispiele für am meisten bevorzugte Amine sind 1,5-Diaminonaphthalin (NDA), 1,4-Diaminobenzol (pPDA), Bis(p-aminocyclohexyl)methan (PACM), 1,3-Bis(aminomethyl)benzol (m-XDA, Bis(aminomethyl)norbornan und Mischungen davon.

Statt der Amine können auch deren Salze, insbesondere die Hydrochloride oder Carbaminate, vorzugsweise die Hydrochloride der Amine phosgeniert werden. Diese Salze werden dann in der Regel in einem ersten Schritt in situ bei niedriger Temperatur hergestellt, wie beispielsweise in der DE19510259A1 beschrieben, wobei gegebenenfalls auf das Einleiten des Inertgases verzichtet werden kann. Bevorzugt ist es die Amine direkt zu phosgenieren (Basenphosgenierung) und zwar in einer zweistufigen Reaktion bei unterschiedlicher Reaktionstemperatur (Kalt-Heiß-Phosgenierung).

Die Umsetzung erfolgt in Gegenwart eines Lösemittels. Es sind alle dem Fachmann bekannten Lösemittel geeignet, die unter den vorherrschenden Reaktionsbedingungen inert oder zumindest weitgehend inert sind. Bevorzugte Lösemittel sind ausgewählt aus der Gruppe bestehend aus aromatischen Kohlenwasserstoffen, halogenierten aromatischen Kohlenwasserstoffen, insbesondere chlorierten aromatischen Kohlenwasserstoffen, Estern, Ethern und halogenierten Kohlenwasserstoffen und Mischungen davon. Erfindungsgemäß besonders bevorzugt eingesetzte aromatische Kohlenwasserstoffe sind ausgewählt aus der Gruppe bestehend aus Toluol, Brombenzol, Chlorbenzol, Dichlorbenzol, insbesondere o-Dichlorbenzol, und Mischungen davon. Besonders bevorzugt werden erfindungsgemäß Chlorbenzol, o-Dichlorbenzol oder Mischungen dieser beiden Lösemittel eingesetzt.

Die Umsetzung wird chargenweise durchgeführt. Darunter ist vorliegend zu verstehen, dass das flüssige Umsetzungsprodukt den Reaktoren nicht kontinuierlich entnommen wird, sondern das flüssige Umsetzungsprodukt aus einem Reaktor im Wesentlichen erst dann entnommen und in eine Rohproduktvorlage oder direkt zur weiteren Aufbereitung überführt wird, wenn die Umsetzung zum Isocyanat in diesem Reaktor beendet ist. Dem Fachmann sind verschiedene Methoden bekannt, den Endpunkt der Reaktion zu bestimmen. Meist wird der sogenannte Klarpunkt herangezogen, also der Zeitpunkt, an dem aus der zunächst vorliegenden Suspension bei der Heißphosgenierung eine klare Lösung entstanden ist. Alternativ kann auch das Ende der Entwicklung von HCl-Gas herangezogen werden oder ein konstanter NCO-Gehalt in der Reaktionsmischung. Auch während der Reaktion kann jedoch ein Teil der flüssigen Reaktionsmischung aus dem Reaktor entnommen und wieder in diesen zurückgeführt werden, um beispielsweise eine bessere Durchmischung herbeizuführen oder die Reaktionsmischung zu temperieren, ohne die Bedingung der chargenweisen Phosgenierung im Sinne der vorliegenden Erfindung zu verletzen. Gasförmige Ströme umfassend im Wesentlichen Chlorwasserstoff, Phosgen, Inertgas und Lösemitteldämpfe können den Reaktoren hingegen jederzeit, auch kontinuierlich entnommen und aufbereitet werden. Bevorzugt erfolgt die Umsetzung im sogenannten Semi-Batch-Verfahren, auch als Fed-Batch-Verfahren oder Zulauf-Verfahren bezeichnet, das heißt, Amin und/oder Phosgen und gegebenenfalls Inertgas werden ausgehend von einer teilweisen Befüllung des Reaktors während der Reaktion zugefüttert.

Als Reaktoren eigenen sich insbesondere Rührkessel, aber auch andere Reaktorformen, beispielsweise Schlaufenreaktoren, sind prinzipiell einsetzbar. Erfindungsgemäß erfolgt die Umsetzung von Amin mit Phosgen in mindestens 2 parallel angeordneten Reaktoren, bevorzugt in 2 bis 10 parallel angeordneten Reaktoren, besonders bevorzugt in 2 bis 6 parallel angeordneten Reaktoren und ganz besonders bevorzugt in 2 oder 4 parallel angeordneten Reaktoren. Die Umsetzung erfolgt vorzugsweise bei einem absoluten Druck von 0,100 MPa bis 2,000 MPa, bevorzugt 0,105 MPa bis 1,000 MPa und besonders bevorzugt von 0,110 MPa bis 0,600 MPa. Entsprechend sind die Reaktoren vorzugsweise mit einer Druckhalteeinrichtung, beispielsweise einem Regelventil ausgestattet, über die Prozessabgas aus dem Reaktor entweichen kann.

Bei einer Basenphosgenierung, also der Umsetzung der Amine mit Phosgen, wird die Reaktion vorzugsweise zweistufig in dem inerten Lösemittel durchgeführt. Solche Reaktionen sind beispielsweise in W. Siefken, Liebigs Annalen der Chemie, 562 (1949) S. 96 beschrieben. Im ersten Stadium, der Kalt-Phosgenierung, wird die Temperatur der Reaktionsmischung bevorzugt in einem Bereich ≥ 0 °C und < 100 °C gehalten. Es entsteht eine Suspension, die Carbaminsäurechlorid, Aminhydrochlorid und geringe Mengen an freiem Isocyanat enthält. Bevorzugt wird dabei eine Lösung von Phosgen in einem inerten Lösemittel vorgelegt und dann eine Lösung oder Suspension des Amins im gleichen Lösemittel sowie gegebenenfalls weiteres Phosgen zugegeben. Auf diese Weise wird die Konzentration von freiem Amin gering gehalten und somit die unerwünschte Bildung von Harnstoffen unterdrückt.

Im zweiten Stadium, der Heißphosgenierung, wird die Temperatur erhöht und liegt dann vorzugsweise in einem Bereich von 120 °C bis 200 °C. Dieses zum Beginn der Heißphosgenierung durchgeführte Aufheizen der Reaktionsmischung auf eine Temperatur oberhalb von 100 °C ist vorliegend als Schritt (B) "Übergang in die Heißphosgenierung" zu verstehen. Die Temperatur wird dann vorzugsweise in diesem Bereich gehalten, während vorzugsweise mindestens so lange weiteres Phosgen zugeführt wird, bis die Umsetzung zum Isocyanat beendet ist, also die HCl-Entwicklung zum Erliegen kommt und/oder die Reaktionsmischung aufklart. Phosgen wird für die Reaktion zweckmäßig im Überschuss verwendet. Bei Bedarf kann die Reaktion sowohl in der Kalt- als auch in der Heißphosgenierung unter Einleitung eines inerten Gases durchgeführt werden.

Sofern es sich bei den umzusetzenden Aminen um hochschmelzende und in dem Lösemittel schlecht lösliche Amine handelt, kann auch eine Suspension des Amins zur Phosgenierung eingesetzt werden. Diese wird vorzugsweise durch Dispergierung mit einem dynamischen Mischaggregat hergestellt, wie in EP2897933B1, Absatz [0020] bis Absatz [0024], beschrieben.

Bei der Amin-Hydrochlorid- oder Carbaminat-Phosgenierung wird das Amin bevorzugt zunächst mit Chlorwasserstoffgas oder Kohlendioxid in einem inerten flüssigen Medium zur Erzeugung des entsprechenden Salzes umgesetzt. Die Reaktionstemperatur liegt während dieser Salzbildung vorzugsweise in einem Bereich von 0 bis 80 °C. Anschließend oder zeitgleich kann bereits eine erste Umsetzung mit Phosgen erfolgen. Dann schließt sich ein weiterer Phosgenierschritt an, der im Wesentlichen der Heiß-Phosgenierung aus der oben beschriebenen Basenphosgenierung ähnelt und deshalb im Folgenden ebenfalls als Heißphosgenierung bezeichnet wird. Es wird also auch hier die Temperatur bevorzugt im Bereich von 120 bis 200 °C gehalten und währenddessen vorzugsweise Phosgen und optional ein Inertgas in die Reaktionsmischung eingeleitet. Die Einleitung erfolgt vorzugsweise so lange, bis die Umsetzung zum Isocyanat beendet ist. Auch hier wird Phosgen vorzugsweise im Überschuss eingesetzt, um die Reaktion zu beschleunigen.

Die Phosgenierung erfolgt üblicherweise mit einem stöchiometrischen Überschuss an Phosgen, das heißt, pro mol Aminogruppen wird mehr als ein mol Phosgen eingesetzt. Bevorzugt beträgt das molare Verhältnis von insgesamt eingesetztem Phosgen zu Aminogruppen 1,02:1 bis 20,0:1, besonders bevorzugt 1,1:1 bis 10,0:1 und ganz besonders bevorzugt 1,2:1 bis 5,0:1. Gegebenenfalls kann dem Reaktionsgemisch während der Umsetzung weiteres Phosgen oder Phosgenlösung zugeführt werden, um einen ausreichenden Phosgenüberschuss aufrecht zu erhalten bzw. um einen Verlust an Phosgen auszugleichen.

Sowohl bei der Basenphosgenierung als auch bei der Amin-Hydrochlorid- bzw. der Carbaminat-Phosgenierung wird nach Beendigung der Reaktion das verbliebene Phosgen und Chlorwasserstoffgas bevorzugt mit einem Inertgas, vorzugsweise mit Stickstoff, ausgeblasen. Bei Bedarf kann eine Filtration der Reaktionsmischung erfolgen, um gegebenenfalls vorhandene Feststoffe wie beispielsweise nicht umgesetzte Amin-Hydrochloride zu entfernen.

Im Verlauf der chargenweisen Phosgenierung ergeben sich für jede Charge in der Regel mindestens zwei Maxima in Bezug auf den Massenstrom an Prozessabgas. Ein erhöhter Massenstrom an Prozessabgas aus einem Reaktor tritt beispielsweise beim Übergang in die Heißphosgenierung auf. Durch die Erwärmung der Reaktionsmischung sinkt die Löslichkeit von Gasen in der Mischung und es kommt zum Ausgasen gelöster Gase und somit zu einem erhöhten Abgasstrom aus dem Reaktor, der je nach gewähltem Verfahren insbesondere Phosgen, Chlorwasserstoff und/oder Kohlendioxid umfasst. Ein zweites Maximum tritt nach Beendigung der Reaktion auf, wenn verbliebenes Phosgen und Chlorwasserstoff aus der Reaktionsmischung ausgetrieben werden und, sofern die Reaktion unter Druck erfolgte, der Reaktor entspannt wird.

Um eine ökonomischere Auslegung der Apparate und Betriebsbedingungen für die Prozessabgasaufbereitung zu ermöglichen, werden erfindungsgemäß mindestens zwei der Reaktoren asynchron zueinander betrieben, das heißt, dass wenigstens ein Reaktor asynchron zu wenigstens einem der anderen Reaktoren betrieben wird. Dies bedeutet, dass der Schritt A) *Übergang in die Heißphosgenierung,* also das Aufheizen der Reaktionsmischung auf über 100 °C und/oder der Schritt B) *Austreiben von verbliebenem Phosgen und*/*oder Chlorwasserstoff,* welches auch gegebenenfalls das Entspannen des Reaktors auf einen niedrigeren Druck umfasst, in diesem wenigstens einen Reaktor mit zeitlichem Versatz oder unter anderen Bedingungen, insbesondere mit einer anderen Geschwindigkeit, erfolgt als in wenigstens einem der anderen Reaktoren. Es ist beispielsweise beim Übergang in die Heißphosgenierung möglich, in den mindestens zwei asynchron betriebenen Reaktoren die gleiche Temperaturrampe zeitversetzt zu vollziehen oder aber in wenigstens einem Reaktor eine andersartige, insbesondere flachere Temperaturrampe mit oder ohne Zeitversatz zu vollziehen, so dass das damit verbundene Maximum des Abgasmassenstroms geringer ausfällt und/oder nicht mit dem Maximum der Abgasmassenströme der anderen Reaktoren zusammenfällt. Besonders bevorzugt vollziehen die asynchron betriebenen Reaktoren die gleiche Temperaturrampe mit einem zeitlichen Versatz, so dass die Temperaturerhöhung im Übergang zu der Heißphosgenierung nicht zeitgleich erfolgt. Vorzugsweise beträgt der zeitliche Versatz mindestens 5 Minuten, besonders bevorzugt mindestens 10 Minuten und ganz besonders bevorzugt mindestens 20 Minuten. Ebenfalls vorzugsweise beträgt der zeitliche Versatz maximal 24 h, bevorzugt maximal 8 Stunden, besonders bevorzugt maximal 2 h und ganz besonders bevorzugt maximal 1 h. Sofern sich die Zeiträume in denen die Temperaturerhöhungen in den mindestens zwei der parallel angeordneten Reaktoren, in denen der Übergang in die Heißphosgenierung asynchron erfolgt, überlappen, ist der Zeitpunkt maßgeblich, an dem jeweils die Temperatur von 100 °C in der Reaktionsmischung überschritten wird und die genannten zeitlichen Versätze beziehen sich auf diesen Zeitpunkt.

Entsprechend ist es auch bei Schritt B) dem *Austreiben von verbliebenem Phosgen und*/*oder Chlorwasserstoff.* Hier können beispielsweise die Entspannung und/oder das optionale Einleiten von Inertgas in mindestens zwei der parallel angeordneten Reaktoren asynchron, also zeitversetzt und/oder mit unterschiedlicher Geschwindigkeit erfolgen, um wiederum zu erreichen, dass das damit verbundene Maximum des Abgasmassenstroms geringer ausfällt und/oder nicht mit dem Maximum der Abgasmassenströme der anderen Reaktoren zusammenfällt. Besonders bevorzugt erfolgt die Entspannung und/oder das optionale Einleiten von Inertgas mit den gleichen Vorgaben bezüglich der zeitlichen Druckprofile bzw. Inertgas-Massenströme wie für die anderen Reaktoren, jedoch mit einem zeitlichen Versatz. Vorzugsweise erfolgt das Austreiben von Phosgen und/oder Chorwasserstoff mit einem zeitlichen Versatz von mindestens 5 Minuten, besonders bevorzugt mindestens 10 Minuten und ganz besonders bevorzugt mindestens 20 Minuten. Ebenfalls vorzugsweise beträgt der zeitliche Versatz maximal 24 h, bevorzugt maximal 8 Stunden, besonders bevorzugt maximal 2 h und ganz besonders bevorzugt maximal 1 h.

In einer weiteren bevorzugten Ausführungsform der Erfindung werden die mindestens zwei der parallel angeordneten Reaktoren, in denen Schritt A) und/oder Schritt B) asynchron erfolgen, gemäß der gleichen Fahranweisung betrieben, durchlaufen die einzelnen Prozessschritte jedoch mit einem zeitlichen Versatz von mindestens 5 Minuten, bevorzugt mindestens 10 Minuten und besonders bevorzugt mindestens 20 Minuten.

In einer weiteren bevorzugten Ausführungsform werden mindestens drei, bevorzugt mindestens vier und besonders bevorzugt alle der parallel angeordneten Reaktoren asynchron zueinander betrieben. Bevorzugt werden alle Reaktoren gemäß der gleichen Fahranweisung betrieben, durchlaufen jedoch die einzelnen Prozessschritte mit einem zeitlichen Versatz zueinander. Vorzugsweise beträgt der zeitliche Versatz mindestens 5 Minuten, besonders bevorzugt mindestens 10 Minuten und ganz besonders bevorzugt mindestens 20 Minuten. Ebenfalls vorzugsweise beträgt der zeitliche Versatz maximal 24 h, bevorzugt maximal 8 Stunden, besonders bevorzugt maximal 2 h und ganz besonders bevorzugt maximal 1 h.

Weiterhin ist es ebenfalls vorteilhaft, die Reaktoren derart zu takten, dass nicht einer der Reaktoren sich im Übergang zur Heißphosgenierung befindet, also eine Temperaturerhöhung auf über 100 °C durchläuft, während in einem anderen Reaktor gerade Phosgen und Chlorwasserstoff ausgetrieben werden, bzw. der Reaktor entspannt wird.

Das Prozessabgas enthält in der Regel Phosgen, Chlorwasserstoff, Lösemitteldämpfe sowie gegebenenfalls Inertgas und Spuren von anderen flüchtigen Komponenten der Reaktionsmischung wie beispielsweise Aminen oder Isocyanaten. In einer weiteren bevorzugten Ausführungsform werden aus den Reaktoren Prozessabgasströme entnommen und zumindest teilweise zu wenigstens einem Prozessabgasstrom vereinigt, aus dem Phosgen zurückgewonnen wird. Besonders bevorzugt wird das zurückgewonnene Phosgen wieder zur Umsetzung von Amin zum Isocyanat eingesetzt. Vorzugsweise werden die Prozessabgasströme der verschiedenen Reaktoren in einer Sammelleitung zusammengeführt und dann gemeinsam einer Aufbereitung zugeführt, in der Phosgen aus dem gesammelten Prozessabgas zurückgewonnen wird.

In einer bevorzugten Ausführungsform der Erfindung umfasst die Rückgewinnung von Phosgen aus dem wenigstens einen Prozessabgasstrom mindestens einen Absorptionsschritt, in dem das Phosgen mit Hilfe mindestens eines Absorbens in einer oder mehrerer Absorptionsvorrichtungen aus dem Prozessabgasstrom unter Erhalt eines Abgasstroms und einer Phosgenlösung ausgewaschen wird. Hierbei wird das Prozessabgas mit einem Absorbens in Kontakt gebracht, um Phosgen aus dem Prozessabgas auszuwaschen und einen möglichst phosgenarmen Abgasstrom enthaltend Chlorwasserstoff zu erzeugen.

Im Allgemeinen kann ein solcher Absorptionsschritt in einer oder mehrerer geeigneter Absorptionsvorrichtungen erfolgen.

Geeignete Absorptionsvorrichtungen sind beispielsweise Gaswäscher, bei denen der Prozessabgasstrom mit einem Flüssigkeitsstrom, auch Absorbens oder Waschmedium genannt, in Kontakt gebracht wird, wie beispielsweise Tauchwäscher, Sprühwäscher, Füllkörperkolonnen, Packungskolonnen, Bodenkolonnen oder Fallfilmabsorber. Für eine effiziente Absorption des Phosgens aus dem Prozessabgasstrom können auch verschiedenartige Gaswäscher kombiniert werden. Dabei ist es bevorzugt zwei oder mehr, besonders bevorzugt zwei Gaswäscher in einer Absorptionsvorrichtung zu kombinieren. Vorzugsweise umfasst die Absorptionsvorrichtung einen Gaswäscher ausgewählt aus der Gruppe bestehend aus Füllkörperkolonnen, Packungskolonnen und Bodenkolonnen, bevorzugt bestehend aus Füllkörperkolonnen und Packungskolonnen.

Eine besonders geeignete Absorptionsvorrichtung umfasst einen Fallfilmabsorber, der geeignet ist, eine isotherme Absorption durchzuführen und eine Boden-, Füllkörper- oder Packungskolonne, bevorzugt eine Füllkörper- oder Packungskolonne, besonders bevorzugt eine Packungskolonne, die geeignet ist, eine adiabate Absorption durchzuführen. Bei dem Fallfilmabsorber handelt es sich vorzugsweise um einen vertikal angeordneten Rohrbündelwärmetauscher, wobei das Prozessabgas und das Absorbens sowohl auf der Rohr- als auch auf der Mantelseite des Wärmetauschers, vorzugsweise auf der Rohrseite des Wärmetauschers geführt werden, während auf der jeweils anderen Seite ein Kühlmedium im Gleich- oder im Gegenstrom, vorzugsweise im Gegenstrom zu dem Prozessabgasstrom geführt wird. Das Prozessabgas und das Absorbens können ebenfalls im Gleich- oder im Gegenstrom zueinander, vorzugsweise im Gegenstrom zueinander geführt werden. Die Rohre können optional zur Verbesserung des Stoff- und/oder Wärmeaustauschs mit Füllkörpern, Packungen oder anderen Einbauten versehen werden. Die Boden-, Füllkörper- oder Packungskolonne ist vorzugsweise derart eingerichtet, dass das Prozessabgas aus dem isothermen Gaswäscher im Gegenstrom mit dem Absorbens kontaktiert wird. Das Absorbens wird vorzugsweise am oberen Ende der Kolonne über einen Flüssigkeitsverteiler aufgegeben. Bevorzugt handelt es sich um eine Packungskolonne mit einer strukturierten Packung. Besonders bevorzugt werden der Fallfilmabsorber und die Boden-, Füllkörper oder Packungskolonne in einem kolonnenförmigen Apparat kombiniert, der am unteren Ende eine Zuleitung für das Prozessabgas umfasst. Darüber ist der Fallfilmabsorber angeordnet und über diesem die Boden-, Füllkörper- oder Packungskolonne. Oberhalb des Fallfilmabsorbers sowie am Kopf des kolonnenförmigen Apparats befinden sich vorzugsweise Flüssigkeitsverteiler um das Absorbens gleichmäßig auf die Rohre bzw. die Kolonne zu verteilen. Am Kopf der Absorptionsvorrichtung befindet sich optional ein innen- oder außenliegender Kondensator zur weiteren Kühlung des nun an Phosgen abgereicherten Abgasstroms.

Der möglichst phosgenarme Abgasstrom besteht üblicherweise im Wesentlichen, also zu mindestens 80 Vol.-%, bevorzugt zu mindestens 95 Vol.-% und besonders bevorzugt zu mindestens 98 Vol.-% aus Chlorwasserstoff. Er enthält darüber hinaus Reste des Lösemittels, gegebenenfalls Inertgase und Spuren von Phosgen. Der Phosgengehalt dieses Abgasstroms liegt vorzugsweise bei maximal 1,0 Vol.-%, besonders bevorzugt bei maximal 0,5 Vol.-% und ganz besonders bevorzugt maximal 0,2 Vol-%. Die natürliche und grundsätzlich erstrebenswerte Untergrenze für den Phosgengehalt in diesem Abgasstrom ist 0,00 Vol-%. Das Einhalten dieser Grenze kann aber einerseits zu einem erhöhten Verbrauch an Absorbens führen und andererseits kann es für den Aufbau einer Regelung vorteilhaft sein, einen geringen Phosgengehalt im Abgas zuzulassen, z.B. um diesen als Regelungsgröße zu verwenden. Deshalb liegt der Phosgengehalt im Abgasstrom vorzugsweise bei mindestens 0,01 Vol-%, besonders bevorzugt bei mindestens 0,02 Vol.-% und ganz besonders bevorzugt bei mindestens 0,05 Vol-%.

Um eine möglichst effiziente Absorption zu erreichen, ist es vorteilhaft, das Prozessabgas zunächst auf eine Temperatur im Bereich von 5 °C bis -25 °C bevorzugt im Bereich von 0 °C bis -20 °C, besonders bevorzugt im Bereich von -5 °C bis -15 °C abzukühlen. Die Abkühlung kann einfach mittels eines oder mehrerer Wärmetauscher erfolgen oder durch Quenchen, also indem das Prozessabgas mit einer bereits gekühlten Flüssigkeit in einer Quench-Vorrichtung kontaktiert wird. Dies kann beispielsweise in einem Gaswäscher, vorzugsweise einem Sprühwäscher, oder in einem berieselten Wärmetauscher erfolgen. Als gekühlte Flüssigkeit eignet sich beispielsweise das Absorbens. Vorzugsweise wird das Absorbens dabei nach dem Kontakt mit dem Prozessgas als Flüssigkeit abgeschieden und diese Flüssigkeit in die Quench-Vorrichtung rezirkuliert, wobei der Flüssigkeitskreislauf oder die Quenchvorrichtung selbst einen Kühler enthält, um die Flüssigkeit auf die gewünschte Temperatur abzukühlen. Während der Abkühlung kommt es zur teilweisen Kondensation bzw. Absorption von Phosgen.

Die verbleibende Gasphase wird dann, vorzugsweise im Gegenstrom, mit dem Absorbens in Kontakt gebracht. Dabei wird bevorzugt die aufsteigende Gasphase im Gegenstrom mit dem absteigenden flüssigen Absorbens kontaktiert. Geeignete Vorrichtungen hierfür sind dem Fachmann bekannt. Vorzugsweise handelt es sich bei der Absorption um eine Kombination aus zwei Absorptionsschritten, wobei das Gas bevorzugt zunächst eine isotherme Absorption und dann eine adiabate Absorption durchläuft. Der Mengenstrom des Absorbens ist vorzugsweise variabel und kann jeweils an die vorherrschenden Prozessbedingungen und die daraus resultierenden Erfordernisse angepasst werden. Als Absorbens wird vorzugsweise das gleiche Lösemittel eingesetzt, das auch während der Umsetzung des Amins oder dessen Salzes mit Phosgen zugegen ist. Besonders bevorzugt wird als Absorbens Chlorbenzol, o-Dichlorbenzol oder eine Mischung aus beiden verwendet. In der bevorzugten Ausführungsform mit zwei Absorptionsschritten wird frisches oder aufbereitetes Absorbens bevorzugt im zweiten Absorptionsschritt, also dem Absorptionsschritt, den das Gas später durchläuft, eingebracht, während im ersten Absorptionsschritt bevorzugt bereits beladenes Absorbens aus dem zweiten Absorptionsschritt, gegebenenfalls zusammen mit frischem Absorbens und/oder einem zurückgeführten, ebenfalls bereits beladenen Absorbens aus dem ersten Absorptionsschritt und/oder dem vorausgehenden Abkühlvorgang genutzt wird. Bevorzugt durchläuft der Gasstrom nach dem Kontakt mit dem Absorbens einen Kondensator, um aufgenommenes Absorbens zu kondensieren und möglichst vollständig aus dem Gasstrom abzutrennen.

Das beladene Absorbens kann, wie zuvor beschrieben, als Quench-Medium eingesetzt werden, um den Prozessabgasstrom zunächst abzukühlen. Ein Teil des Absorbens wird entweder kontinuierlich oder diskontinuierlich entnommen und, gegebenenfalls nach Zugabe von weiterem Phosgen und/oder Lösemittel, wieder zur Isocyanat-Herstellung, bevorzugt in einem der Reaktoren zur Phosgenierung des Amins oder dessen Salzes, eingesetzt. Bevorzugt handelt es sich bei dem entnommenen, beladenen Absorbens um eine Lösung enthaltend 20 Gew.-% bis 70 Gew.-% , besonders bevorzugt 30 Gew.-% bis 68 Gew.-% und ganz besonders bevorzugt 42 Gew.-% bis 66 Gew.-% Phosgen in Chlorbenzol. Der Gehalt der Lösung hängt dabei insbesondere von dem Phosgengehalt des Prozessabgases, von der Menge an frischem Absorbens zur Absorption und der Menge an entnommenem Absorbens ab.

In einer besonders bevorzugten Ausführungsform des erfindungsgemäßen Verfahrens wird der Massenstrom an Absorbens in die Absorptionsvorrichtung vorzugsweise automatisch gesteuert oder bevorzugt geregelt. Hierzu wird ein Sollwert für den Massenstrom an Absorbens in die Absorptionsvorrichtung ermittelt und in eine Stellgröße für einen Aktor übersetzt, der den Massenstrom beeinflusst. In die Ermittlung des Sollwertes für den Massenstrom fließt dabei mindestens eine der folgenden Messgrößen a) bis d), beziehungsweise deren Veränderung über die Zeit ein:
a) der Phosgengehalt des nach dem mindestens einen Absorptionsschritt erhaltenen Abgasstroms
b) der Massenstrom an Prozessabgas in die Absorptionsvorrichtung
c) die Konzentration der im Absorptionsschritt erhaltenen Phosgenlösung
d) der Gehalt an Chlorwasserstoff im Prozessabgas-Strom in die Absorptionsvorrichtung

Eine solche Vorgehensweise bietet sich insbesondere bei chargenweiser Reaktionsführung an, wobei es zwangsläufig zu Schwankungen der Prozessabgasmenge und -zusammensetzung kommt. Aus dem Stand der Technik ist die Empfehlung bekannt, die Menge des Absorbens am Massenstrom des Phosgens zur Absorptionsvorrichtung auszurichten. So sind beispielsweise in DE102008009761 A1, Absatz [0048], oder in US2007/0249859 A1, Absatz [0048], Gewichtsverhältnisse von Lösemittel (vorliegend Absorbens) und Phosgen am Eintritt in die Absorption von 0,1:1 bis 10:1, vorzugsweise 1:1 bis 3:1 offenbart. Wie mit Schwankungen der Phosgenmenge und des Gesamtmassenstroms an Prozessabgas am Eintritt der Absorption umzugehen ist, wird nicht weiter thematisiert, da diese Dokumente sich vornehmlich mit kontinuierlichen Prozessen befassen, bei denen derartige Schwankungen nicht oder nur in deutlich geringerem Maße auftreten. In der betrieblichen Praxis hat sich jedoch gezeigt, dass bei alleiniger Anwendung dieses Kriteriums, insbesondere im chargenweisen Betrieb eines einzelnen Reaktors oder einer Mehrzahl synchron betriebener Reaktoren nicht ausreichend ist, um den Prozess zufriedenstellend effektiv und effizient zu betreiben. Es kommt im chargenweisen Betrieb immer wieder zum Durchschlagen von Phosgen in den chlorwasserstoffhaltigen Abgasstrom nach der Absorption. Auch eine Auslegung der Absorptionsvorrichtung auf die Spitzenlast ist problematisch, da dann in Zeiten geringerer Last weiterhin eine hohe Menge Absorbens aufgegeben werden müsste, um eine ausreichende Flüssigkeitsbelastung für einen guten Stoffaustausch aufrecht zu erhalten. Das wiederum führt zu starken Schwankungen in der Konzentration des Phosgens im Absorbens und erschwert in der Folge die Wiederverwendung der gewonnenen Phosgenlösung in der Phosgenierungsreaktion bzw. erhöht den Aufwand für die Aufbereitung des Absorbens.

Um eine möglichst konstante Qualität des phosgenarmen Abgasstroms nach der Absorption zu erreichen, ist es bevorzugt, den Phosgengehalt des nach dem mindestens einen Absorptionsschritt erhaltenen Abgasstroms in die Ermittlung des Sollwerts für den Massenstrom an Absorbens einzubeziehen. Dies kann beispielsweise erfolgen, indem der Gehalt an Phosgen in dem genannten Abgasstrom bestimmt wird und mit einem Zielwert verglichen wird. Liegt der Gehalt oberhalb des Zielwerts, so wird der Sollwert für den Massenstrom an Absorbens zur Absorptionsvorrichtung erhöht. Liegt der Gehalt unterhalb des Zielwerts, so wird der Massenstrom an Absorbens zur Absorptionsvorrichtung gesenkt. Methoden zur Bestimmung des Phosgengehalts im Abgasstrom sind dem Fachmann bekannt. Die Bestimmung kann beispielsweise mittels IR-Filter-Fotometrie, beispielsweise mit einem Mehrkomponenten-Prozessfotometer erfolgen.

Ein weiterer Gegenstand der Erfindung ist daher ein computerimplementiertes Verfahren zur Anpassung eines Massenstroms an Absorbens in einer Absorptionsvorrichtung für die Aufarbeitung Phosgen enthaltender Prozessabgasströme, bei dem ein Sollwert für den Massenstrom an Absorbens ermittelt und in eine Stellgröße für einen Aktor übersetzt wird, der den Massenstrom beeinflusst, wobei in die Ermittlung des Sollwertes für den Massenstrom mindestens eine der folgenden Messgrößen, beziehungsweise deren Veränderung über die Zeit einfließt:
- der Phosgengehalt des nach dem mindestens einen Absorptionsschritt erhaltenen Abgasstroms
- der Massenstrom an Prozessabgas in die Absorptionsvorrichtung
- die Konzentration der im Absorptionsschritt erhaltenen Phosgenlösung
- der Gehalt an Chlorwasserstoff im Prozessabgasstrom in die Absorptionsvorrichtung.

In einer bevorzugten Ausführungsform des computerimplementierten Verfahrens wird der Phosgengehalt des nach dem mindestens einen Absorptionsschritt erhaltenen Abgasstroms in die Ermittlung des Sollwerts für den Massenstrom an Absorbens einbezogen.

### Beispiele

### Vergleichsbeispiel 1:

In zwei parallel installierten 0,5 m³ Rührkesselreaktoren mit Temperiervorrichtung und aufgesetztem Rückflusskühler wurde jeweils bei 0° C eine Lösung von 60 kg Phosgen in 150 kg Chlorbenzol hergestellt. Das Abgas aus den Reaktoren wurde nach dem Durchlaufen der Rückflusskühler in einer Sammelleitung zusammengeführt. Unter Rühren wurde in beiden Reaktoren gleichzeitig zu der Phosgenlösung jeweils eine Suspension von 25 kg 1,5-Diaminonaphthalin in 75 kg Chlorbenzol zugegeben. Ohne weitere Kühlung wurde die Suspension für ca. 120 min gerührt, bevor der Übergang von der Phase der Kaltphosgenierung in die Phase der Heißphosgenierung erfolgte. Dazu wurde die Reaktionsmischung in beiden Reaktoren gleichzeitig erhitzt und etwa 6 h bei Rückfluss gehalten. Der Druck während der Reaktion betrug ca. 2,9 bar(a). Nach dem Aufklaren der Reaktionsmischung, das das Ende der Phosgenier-Rekation anzeigt, wurden die Reaktoren zunächst entspannt und das flüssige Rohprodukt wurde abgelassen und das Isocyanat wurde in einer Destillationssequenz isoliert.

Das Abgas aus der Sammelleitung wurde währenddessen einer zweistufigen Absoprtionskolonne zugeführt, in der es im Gegenstrom mit Chlorbenzol gewaschen wurde. Das Prozessabgas durchlief dabei zunächst eine isotherme Absorptionsstufe bei ca. -15 °C und anschließend eine adiabate Absorptionsstufe, bevor es als Abgas den Prozess verließ. Frisches Chlorbenzol wurde mit -5 °C am oberen Ende der adiabaten Absorptionsstufe aufgegeben. Der Massenstrom an frischem Chlorbenzol zur Absorptionskolonne betrug ca. 36 kg/h. Nach dem Durchlaufen der adiabaten Absorptionsstufe wurde die dort erhaltene Lösung aus Phosgen in Chlorbenzol als Waschlösung in die isotherme Absorptionsstufe weitergeleitet, so dass letztlich am Sumpf der zweistufigen Absorptionskolonne eine Lösung von Phosgen in MCB entnommen wurde, die nach Anpassen der Phosgenkonzentration als Ausgangsmaterial für weitere Phosgenierungen diente. Die Konzentration der entnommenen Phosgenlösung fiel im Laufe der Reaktion ab, so dass für die Anpassung der Phosgenkonzentration für den erneuten Einsatz eine steigende Menge frisches Phosgen zugesetzt werden musste. Am Kopf der Absorptionskolonne wurde ein weitgehend von Phosgen befreiter Chlorwasserstoffstrom erhalten. Kurzzeitig kam es jedoch während der Reaktion zum Durchschlag einer geringen Menge an Phosgen in das Abgas der Absorptionsvorrichtung, so dass ein erhöhter Aufwand zur weiteren Behandlung des Prozessabgases erforderlich war.

Während des Produktionslaufs wurde in der Sammelleitung ein maximaler Prozessabgasmassenstrom von ca. 70 kg/h beobachtet. Dieser trat beim Übergang von der Phase der Kalt- in die Heißphosgenierung auf und das Prozessabgas bestand zu ca. 90% aus Phosgen und zu ca. 10% aus Chlorwasserstoff. Nach dem Durchlaufen dieses Maximums fiel der Gesamtmassenstrom an Prozessabgas ab und die Zusammensetzung verschob sich hin zu höheren Chlorwasserstoffgehalten. Am Ende der Reaktion kam der Prozessabgasstrom zum Erliegen. Lediglich beim Entspannen der Reaktoren trat noch einmal für kurze Zeit ein erhöhter Massenstrom an Prozessabgas in der Sammelleitung auf, der jedoch weit weniger ausgeprägt war als das erste Maximum.

### Vergleichsbeispiel 2:

Die Synthese aus Vergleichsbeispiel 1 wurde unter gleichen Reaktionsbedingungen wiederholt. Anders als in Vergleichsbeispiel 1 wurde die Absorptionsvorrichtung hier nicht mit einem konstanten Massenstrom an Chlorbenzol beaufschlagt. Dieser wurde stattdessen in Abhängigkeit vom Phosgenmassenstrom im Prozessabgas variiert, so dass der Massenstrom an frischem Chlorwasserstoff in die Absorptionskolonne stets dem 1,3-fachen des Massenstroms an Phosgen in die Absorptionskolonne entsprach, sofern nicht die Mindest-Berieselungsdichte der Absorptionskolonne unterschritten wurde. So betrug der maximale Massenstrom an Chlorbenzol beim Übergang von der Kalt- in die Heißphosgenierung, also zum Zeitpunkt des größten Phosgenmassenstroms im Prozessabgas, 82 kg/h. Während der Heißphosgenierung ungefähr 1 h nach dem Auftreten des Maximums im Prozessabgasmassenstrom, kam es zu einem Durchbruch von Phosgen in das Abgas der Absorptionsvorrichtung, der sich in den folgenden 1,5 h verstärkte. Über die Gesamtlaufzeit der Batch-Produktion war der Verbrauch an Chlorbenzol höher als in Beispiel 1, so dass sich im Mittel eine geringere Konzentration der am Sumpf der Kolonne erhaltenen Phosgenlösung einstellte. Außerdem wurde ein stärkerer Durchbruch von Phosgen in das Abgas der Absorptionskolonne als in Vergleichsbeispiel 1 festgestellt.

### Beispiel 1 (Erfindungsgemäß):

Die Synthese aus Beispiel 1 wurde wiederholt mit dem Unterschied, dass der zweite Reaktor mit einem Zeitversatz von 3 Stunden betrieben wurde, das heißt, dass die Zugabe der Amin-Suspension ebenso wie das Aufheizen zum Übergang von der Kaltphosgenierung in die Heißphosgenierung im zweiten Reaktor nicht synchron mit dem ersten Reaktor erfolgten sondern mit eben diesem Zeitversatz von 3 Stunden. Des Weiteren wurde der Massenstrom an frischem Chlorbenzol zur Absorptionskolonne auf konstante 30 kg/h eingestellt.

Beim Übergang des ersten Reaktors von der Kalt- in die Heißphosgenierung wurde ein erstes Maxiumum des Prozessabgasmassenstroms in der Sammelleitung beobachtet. Der Massenstrom betrug zu diesem Zeitpunkt ca. 35 kg/h und das Prozessabgas bestand wiederum zu ca. 90% aus Phosgen und zu ca. 10% aus Chlorwasserstoff. Im weiteren Verlauf der Produktion durchlief der Prozessabgasmassenstrom ein zweites, höheres Maximum von ca. 46 kg/h. Dieses trat beim Übergang des zweiten Reaktors von der Phase der Kalt- in die Heißphosgenierung auf und das Prozessabgas bestand zu ca. 79% aus Phosgen und zu ca. 21% aus Chlorwasserstoff. Nach dem Durchlaufen dieses Maximums fiel der Gesamtmassenstrom an Prozessabgas ab und die Zusammensetzung verschob sich hin zu höheren Chlorwasserstoffgehalten. Da auch das Entspannen der beiden Reaktoren nach der Reaktion mit einem entsprechenden Zeitversatz erfolgte, zeigten sich auch im Prozessabgasmassenstrom in der Sammelleitung nicht ein, sondern zwei weitere kurze und weniger ausgeprägte Spitzen.

Trotz des gegenüber Vergleichsbeispiel 1 und Vergleichsbeispiel 2 verringerten Einsatzes von Chlorbenzol in der Absorptionskolonne wurde in diesem Versuch zu keiner Zeit ein Durchbruch von Phosgen in das Abgas der Absorptionskolonne beobachtet. Der Gehalt an Phosgen in der am Sumpf der Absorptionskolonne entnommenen Phosgenlösung schwankte zwar, war aber im Mittel höher als in den zuvor beschriebenen Beispielen 1 und 2. Sowohl die maximale Gasbelastung als auch die Flüssigkeitsbelastung der Absorptionskolonne war gegenüber den Beispielen 1 und 2 reduziert, so dass eine Absorptionskolonne mit geringerem Durchmesser und damit verbunden auch geringeren Investitionskosten eingesetzt werden kann.

### Beispiel 2 (Erfindungsgemäß):

Die Synthese aus Beispiel 1 mit zeitlich versetztem Betrieb der Reaktoren wurde wiederholt. Anders als in Beispiel 1 wurde die Absorptionsvorrichtung hier jedoch nicht mit einem konstanten Massenstrom an Chlorbenzol beaufschlagt. Stattdessen wurde der Phosgengehalt im Abgas aus der Absorptionsvorrichtung mittels einer Online-Messung verfolgt. Der Zielwert für den Phosgengehalt wurde auf 0,05 Vol-% gesetzt und die Abweichung des gemessenen Werts von diesem Zielwert wirkte über einen Regelkreis letztlich auf ein Regelventil in der Zuleitung des frischen Chlorbenzols, um den Chlorbenzol-Massenstrom zur Absorptionskolonne entsprechend anzupassen und die Abweichung so gering wie möglich zu halten.

Über die Gesamtlaufzeit der Batch-Produktion war der Verbrauch an Chlorbenzol in diesem Beispiel noch einmal geringer als in Beispiel 1, so dass sich im Mittel eine höhere Konzentration der am Sumpf der Kolonne erhaltenen Phosgenlösung einstellte während das Abgas aus der Absorptionskolonne über die gesamte Laufzeit nur minimal mit Phosgen belastet war. Der Gehalt an Phosgen war dabei gering und konstant, so dass eine weitere Nachbehandlung zur Vernichtung des Restphosgens einfach gelingt.

## Patentansprüche

1. Verfahren zur Herstellung von Isocyanaten durch Umsetzung von mindestens einem Amin oder dessen Salz mit einem stöchiometrischen Überschuss an Phosgen in kondensierter Phase in Gegenwart eines Lösemittels, umfassend die Schritte:
A) Übergang in eine Heißphosgenierung und
B) Austreiben von verbliebenem Phosgen und/oder Chlorwasserstoff,
**dadurch gekennzeichnet, dass** die Umsetzung chargenweise in mindestens zwei parallel angeordneten Reaktoren erfolgt und dass Schritt A) und/oder Schritt B) in mindestens zwei der parallel angeordneten Reaktoren asynchron erfolgen.

2. Verfahren gemäß Anspruch 1, **dadurch gekennzeichnet, dass** es sich bei dem mindestens einen Amin um mindestens ein Diamin, bevorzugt um 1,5-Diaminonaphthalin (NDA), 1,4-Diaminobenzol (pPDA), 1,5-Diaminopentan (PDA), Bis(p-aminocyclohexyl)methan (PACM), Hexahydrotoluylendiamin (H6TDA), 1,3-Bis(aminomethyl)benzol (m-XDA), Bis(aminomethyl)norbornan (NBDA) oder Mischungen davon handelt.

3. Verfahren gemäß einem der Ansprüche 1 oder 2, **dadurch gekennzeichnet, dass** die Umsetzung im Semi-Batch-Verfahren durchgeführt wird.

4. Verfahren gemäß einem der Ansprüche 1 bis 3, **dadurch gekennzeichnet, dass** die Umsetzung in 2 bis 10, bevorzugt in 2 bis 6 und besonders bevorzugt in 2 bis 4 parallel angeordneten Reaktoren erfolgt.

5. Verfahren gemäß einem der Ansprüche 1 bis 4, **dadurch gekennzeichnet, dass** mindestens drei, bevorzugt mindestens vier und besonders bevorzugt alle der parallel angeordneten Reaktoren asynchron zueinander betrieben werden.

6. Verfahren gemäß einem der Ansprüche 1 bis 4, **dadurch gekennzeichnet, dass** der Schritt A) Übergang in eine Heißphosgenierung in mindestens zwei der Reaktoren asynchron erfolgt.

7. Verfahren gemäß einem der Ansprüche 1 bis 6, **dadurch gekennzeichnet, dass** aus den Reaktoren Prozessabgasströme entnommen und zumindest teilweise zu wenigstens einem Prozessabgasstrom vereinigt werden, aus dem Phosgen zurückgewonnen wird.

8. Verfahren gemäß einem der Ansprüche 1 bis 7, **dadurch gekennzeichnet, dass** aus den Reaktoren Prozessabgasströme entnommen und zumindest teilweise zu wenigstens einem Prozessabgasstrom vereinigt werden, aus dem Phosgen zurückgewonnen wird, wobei die Rückgewinnung von Phosgen aus dem wenigstens einen Prozessabgasstrom mindestens einen Absorptionsschritt umfasst, in dem das Phosgen mit Hilfe mindestens eines Absorbens in einer oder mehrerer Absorptionsvorrichtungen aus dem Prozessabgasstrom unter Erhalt eines Abgasstroms und einer Phosgenlösung ausgewaschen wird.

9. Verfahren gemäß Anspruch 8, **dadurch gekennzeichnet, dass** in dem mindestens einen Absorptionsschritt ein Lösemittel als Absorbens eingesetzt wird, das bevorzugt das Lösemittel ist, welches auch in der Umsetzung des mindestens einen Amins oder dessen Salzes zugegen ist.

10. Verfahren gemäß einem der Ansprüche 8 oder 9, **dadurch gekennzeichnet, dass** die Absorptionsvorrichtung einen Gaswäscher ausgewählt aus der Gruppe bestehend aus Füllkörperkolonnen, Packungskolonnen und Bodenkolonnen, bevorzugt bestehend aus Füllkörperkolonnen und Packungskolonnen, umfasst.

11. Verfahren gemäß einem der Ansprüche 8 bis 10, **dadurch gekennzeichnet, dass** das Absorbens in einem Massenstrom zugegeben wird, dessen Sollwert unter Berücksichtigung mindestens einer der folgenden Messgrößen a) bis d), beziehungsweise deren Veränderung über die Zeit, vorzugsweise automatisch, bestimmt wird:
a) Phosgengehalt des nach dem mindestens einen Absorptionsschritt erhaltenen Abgasstroms,
b) Massenstrom an Prozessabgas in die Absorptionsvorrichtung,
c) Gehalt an Chlorwasserstoff im Prozessabgas-Strom in die Absorptionsvorrichtung
d) Konzentration der im Absorptionsschritt erhaltenen Phosgenlösung.

12. Verfahren gemäß einem der Ansprüche 8 bis 11, **dadurch gekennzeichnet, dass** die in der Absorptionsvorrichtung erhaltene Phosgenlösung, optional nach Zugabe von weiterem Phosgen und/oder Lösemittel, wieder zur Isocyanat-Herstellung eingesetzt wird.

13. Verfahren gemäß einem der Ansprüche 11 oder 12, **dadurch gekennzeichnet, dass** der Phosgengehalt des nach dem mindestens einen Absorptionsschritt erhaltenen Abgasstroms in die Ermittlung des Sollwerts für den Massenstrom an Absorbens einbezogen wird.

14. Computerimplementiertes Verfahren zur Anpassung eines Massenstroms an Absorbens in einer Absorptionsvorrichtung für die Aufarbeitung Phosgen enthaltender Prozessabgasströme, bei dem ein Sollwert für den Massenstrom an Absorbens ermittelt und in eine Stellgröße für einen Aktor übersetzt wird, der den Massenstrom beeinflusst, wobei in die Ermittlung des Sollwertes für den Massenstrom mindestens eine der folgenden Messgrößen, beziehungsweise deren Veränderung über die Zeit einfließt:
- der Phosgengehalt des nach dem mindestens einen Absorptionsschritt erhaltenen Abgasstroms
- der Massenstrom an Prozessabgas in die Absorptionsvorrichtung
- die Konzentration der im Absorptionsschritt erhaltenen Phosgenlösung
- der Gehalt an Chlorwasserstoff im Prozessabgasstrom in die Absorptionsvorrichtung.

15. Verfahren gemäß Anspruch 14, **dadurch gekennzeichnet, dass** der Phosgengehalt des nach dem mindestens einen Absorptionsschritt erhaltenen Abgasstroms in die Ermittlung des Sollwerts für den Massenstrom an Absorbens einbezogen wird.
